# EUROPEAN PATENT SPECIFICATION

(11) **EP 0 601 005 B1**
(45) Date of publication and mention of the grant of the patent: **22.11.2000**
(21) Application number: 92918182.4
(22) Date of filing: 14.08.1992
(51) Int. Cl.: C11D 3/386, C12N 9/58

(54) **DETERGENT ENZYMES**
WASCHMITTELENZYME
ENZYMES POUR DETERGENTS

(30) Priority: 19.08.1991 WO PCT/DK91/00232
(43) Date of publication of application: 15.06.1994
(73) Proprietor: NOVO NORDISK A/S, 2880 Bagsvaerd (DK)
(72) Inventor: PEDERSEN, Kim, Brint, DK-2300 Copenhagen S (DK); CHRISTIANSEN, Margrethe, DK-2930 Klampenborg (DK); LINDEGAARD, Poul, DK-2100 Copenhagen (DK)
(74) Representative: Knudsen, Sten Lottrup
(86) International application number: DK9200242
(87) International publication number: WO9304156

(56) References cited:
- EP-A- 0 335 023
- WO-A-92/18622

## Description

### TECHNICAL FIELD

This invention is in the field of detergent enzymes. More specifically, the invention relates to the use of proteases derived from members of the genus Metarrhizium for detergent purposes.

### BACKGROUND ART

Proteases from Metarrhizium anisopliae (Metschn.) Sorokin have been thoroughly described in the literature.

In "Characterization of Cuticle-Degrading Proteases Produced by the Entomopathogen Metarhizium anisopliae" (R.J. St. Leper. A.K. Charnley, and R.M. Cooper; Archives of Biochemistry and Biophysics (1987) 253, 221-232) a strain (ME1) is described that produces a culture filtrate from which two chymoelastases and three trypsin-like proteases could be detected. Two major alkaline protease components; a chymoelastase (designated Pr1) and a trypsin-like protease (designated Pr2) were purified. Pr1 is characterized by a pI of approximately 10.3, a molecular weight of approximately 25,000, and activity towards e.g. N-succinyl-L-alanyl-L-alanyl-L-prolyl-L-phenylalanyl-p-nitroanilide (Suc-Ala-Ala-Pro-Phe-pNA), hide protein azure, locust cuticle, elastin-Congo Red, and casein. Pr2 is characterized by a pI of approximately 4.42, a molecular weight of approximately 28,500, and activity towards e.g. N-benzoyl-L-phenytalanyl-L-valyl-L-arginyl-p-nitroanilide (Bz-Phe-Val-Arg-pNA), N-benzoyl-L-arginyl-p-nitroanilide, hide protein azure, and casein. Isoenzymes of Pr2 have pI values of 4.0 and 4.2. Pr2 possesses <5% of the cuticle-degrading activity of Pr1, and Pr1 is, therefore, considered to have a greater role in parasitism than Pr2.

In "Distribution of Chymoelastases and Trypsin-like Enzymes in Five Species of Entomopathogenic Deuteromycetes" (R.J. St. Leger. R.M. Cooper, and A.K. Charnley; Archives of Biochemistry and Biophysics (1987) 258, 123-131) four other strains of Metarrhizium anisopliae are mentioned. They all possessed a major chymoelastase (Pr1) with activity towards Suc-Ala-Ala-Pro-Phe-pNA and pI's of approximately 10.4, 10.7, 10.5, and 10.0, and trypsin-like proteases (Pr2) with activity towards Bz-Phe-Val-Arg-pNA and pI's in the acidic range. Two of the isolates M. anisopliae var. major RS 324 and M. anisopliae var. major RS 298 have Pr2 enzymes that require extremely specific substrate sequences, and they are not inhibited by soybean trypsin inhibitor. These highly specific Pr2 enzymes have been claimed in EP Patent Application No. 335,023, where it is mentioned that they may have pharmaceutical, agricultural or Industrial applications.

However, any practical use of the Pr1 enzymes of M. anisopliae has never been suggested.

### SUMMARY OF THE INVENTION

It has now surprisingly been found that alkaline proteases derived from fungi of the genus Metarrhizium possess excellent washing characteristics.

Accordingly, the present invention relates to the use of proteases obtainable from members of the genus Metarrhizium, e.g. from the species M. anisopliae (Metschn.) Sorokin, e.g. the strain DSM No. 6592, or mutants or variants thereof, as detergent enzymes.

In another aspect, the present invention relates to the use of proteases having immunochemical properties identical or partially identical to those of a protease derived from M. anisopliae (Metschn.) Sorokin; DSM No. 6592, as a detergent enzyme.

The invention also provides detergent compositions comprising proteases obtainable from members of the genus Metarrhizium, e.g. from the species M. anisopliae (Metschn.) Sorokin, e.g. the strain DSM No. 6592, or mutants or variants thereof, as well as detergent compositions comprising proteases having immunochemical properties identical or partially identical to those of a protease derived from M. anisopliae (Metschn.) Sorokin; DSM No. 6592.

### DETAILED DISCLOSURE OF THE INVENTION

The present invention relates to the use of proteases derived from fungi of the genus Metarrhizium as detergent enzymes.

### The Organism

The detergent proteases of the invention can be provided from members of the genus Metarrhizium. M. anisopliae (Metschn.) Sorokin is a representative species of this genus. A strain of M. anisopliae (Metschn.) Sorokin, is available from DSM, No. 6592.

### Cultivation of the Microorganism

The microorganisms can be cultivated under aerobic conditions in nutrient media containing assimilable carbon and nitrogen sources together with other essential nutrients, the media being composed in accordance with the principles of the known art. The microorganisms can produce the extracellular proteases of the invention in liquid nutrient media containing commonly used ingredients such as e.g. glucose, starch, dextrins, soybean meal, cotton seed flour, corn steep liquor, and inorganic salts.

### Recovery and Purification of the Proteases

After fermentation, the extracellular proteases produced by the fungi can be recovered and purified according to principles of the known art, e.g. using steps like removal of coarse materials from the culture broth by centrifugation or drum filtration, concentration of the broth by evaporation or ultrafiltration, and purification by preparative isoelectrofocusing, anion-exhange chromatography or affinity chromatography.

### Recombinantly Produced Proteases

The proteases of the invention can also be obtained by recombinant DNA-technology.

A process for the preparation of the protease may comprise isolating a DNA fragment encoding the protease; combining the DNA fragment with an appropriate expression signal in an appropriate plasmid vector; introducing the plasmid vector into an appropriate host either as an autonomously replicating plasmid or integrated into the chromosome; cultivating the host organism under conditions leading to expression of the protease; and recovering the protease from the culture medium. The various steps that constitute this recombinant process are methods known in the art per se.

Preferred host organisms are E coli, or members of the genera Bacillus, Aspergillus, or Streptomyces.

### Assay for Proteolytic Activity

The proteolytic activity is determined with casein as substrate. One Casein Protease Unit (CPU) is defined as the amount of enzyme liberating 1 mM of primary amino groups (determined by comparison with a serine standard) per minute under standard conditions, i.e. incubation for 30 minutes at 25°C and pH 9.5. A folder AF 228, describing the analytical method, is available upon request to Novo Nordisk A/S, Denmark, which folder is hereby included by reference.

### Enzyme

The enzymes of the invention are obtainable from members of the genus Metarrhizium, e.g. from the species M. anisopliae (Metschn.) Sorokin, an example of which is DSM No. 6592.

The proteases of the invention are alkaline proteases with activity towards Suc-Ala-Ala-Pro-Phe-pNA and isoelectric points above 8.0.

A particular protease of the invention is a protease having a pI of approximately 9.2-9.3; a molecular weight of approximately 32,000; being active towards haemoglobin, casein, skim milk, and Suc-Ala-Ala-Pro-Phe-pNA; and having immunochemical properties identical or partially identical to those of a protease derived from M. anisopliae, DSM No. 6592.

### Immunochemical Properties

The immunochemical properties can be determined immunologically by cross-reaction identity tests. The identity tests can be performed by the well-known Oucterlony double immunodiffusion procedure or by tandem crossed immunoelectrophoresis according to N. H. Axelsen; Handbook of Immunoprecipitation-in-Gel Techniques; Blackwell Scientific Publications (1983), chapters 5 and 14. The terms "antigenic identity" and "partial antigenic identity" are described in the same book, chapters 5, 19, and 20.

### Detergent Compositions

According to this invention it has now been found that the proteases mentioned above posses good washing performance.

The detergent composition of the invention may comprise one or more surfactants which may be of an anionic, nonionic, cationic, amphoteric or zwitterionic type, or a mixture of these. Typical examples of anionic surfactants are linear alkyl benzene sulfonates (LAS); alkyl sulfates (AS); alpha olefin sulfonates (AOS); alcohol ethoxy sulfates (AES) and alkali metal salts of natural fatty acids. Examples of nonionic surfactants are alkyl polyethylene glycol ethers; nonylphenol polyethylene glycol ethers; fatty acids esters of sucrose and glucose; and esters of polyethoxylated alkyl glucoside.

The detergent composition of the invention may also contain other detergent ingredients known in the art such as builders, bleaching agents, bleach activators, anti-corrosion agents, sequestering agents, anti soil-redeposition agents, perfumes, stabilizers for the enzymes and bleaching agents, formulations aids, optical brighteners, foam boosters, chelating agents, fillers, fabric softeners, etc. The detergent composition of the invention may be formulated substantially as described in J. Falbe [Falbe. J.; Surfactants in Consumer Products. Theory, Technology and Application; Springer Verlag 1987, vide in particular the section entitled "Frame formulations for liquid/powder heavy-duty detergents"].

It is at present contemplated that the detergent composition of the invention may contain the enzyme preparation in an amount corresponding to 0.0005-0.5 CPU of the proteolytic enzyme per litre of washing liquor.

The detergent compositions of the invention can be formulated in any convenient form, such as powders, liquids, etc.

The detergent composition of the invention may advantageously include one or more other enzymes, e.g. lipases; amylases; cellulases; oxidases; and/or peroxidases, conventionally included in detergent compositions.

The protease of the invention may be included in a detergent composition by adding separate additives containing the detergent protease, or by adding a combined additive comprising different detergent enzymes.

The additive of the invention can be formulated e.g. as granulates, liquids, slurries, etc. Preferred detergent additive formulations are non-dusting granulates, liquids, in particular stabilized liquids, slurries, or protected enzymes. Dust free granulates may be produced according to e.g. GB Patent Specification No. 1,362,365 or US Patent No. 4,106,991, and may optionally be coated by methods known in the art. The detergent enzymes may be mixed before or after granulation. Liquid enzyme preparations may, for instance, be stabilized by adding a polyol such as propylene glycol; a sugar or sugar alcohol; lactic acid or boric acid, according to established methods. Other enzyme stabilizers are well known in the art. Protected enzymes may be prepared according to the method disclosed in EP Patent Application No. 238,216.

The invention is further illustrated in the following examples which are not intended to be in any way limiting to the scope of the invention as claimed.

### EXAMPLE 1

### Cultivation Example

For production of detergent protease the strain M. anisopliae DSM No. 6592 was cultivated at 26°C for 7 days on a rotary shaking table (280 rpm) in 20 500 ml baffled Erlenmeyer flasks containing 100 ml of medium of the following composition (per litre):

| | |
|---|---|
| Pharmamedia | 50 g |
| Dextrin | 30 g |
| MgSO₄,7H₂O | 0.2 g |
| Na₂HPO₄,12H₂O | 8 g |
| KH₂PO₄ | 3 g |
| Pluronic® L-61 | 0.1 ml |
| pH adjusted to 7.0 before autoclaving | |

After cultivation, the enzyme activity of the broth was 9.6 CPU/l.

When the supernatant of a centrifuged sample of the culture broth is subjected to isoelectric focusing on LKB Ampholine® PAG plates (pH 3.5-9.5), followed by applying an overlayer-gel of agarose containing 1% skim milk at pH 8 over the isoelectric focusing gel and incubation for an appropriate time, two major clearing zones caused by proteases can be observed. The major zone occurs at a pI of approximately 9.2. The protease causing this zone has activity towards Suc-Ala-Ala-Pro-Phe-pNA, and no activity towards N-benzoyl-DL-arginyl-p-nitroanilide (BAPNA). The minor zone occurs at a pI of approximately 4.7. The protease causing this zone has activity towards BAPNA and no activity towards Suc-Ala-Ala-Pro-Phe-pNA. The protease composition of this strain, thus, seems similar to the compositions described in the literature, i.e. a trypsin-like protease with a pI in the acidic range and a protease with activity towards Suc-Ala-Ala-Pro-Phe-pNA with a pI in the alkaline range. Additionally, very small clearing zones corresponding to pI's of 7.2, 6.5, 6.0, and 5.7 can be observed after prolonged incubation.

### EXAMPLE 2

### Purification Example

The culture broth obtained in Example 1 was centrifuged in a Beckman J-6 centrifuge at 4000 rpm for 30 minutes.

900 ml supernatant was collected and further purified by anion-exchange chromatography: The supernatant was reacted in a batch process with DEAE-Sephadex A-50 in 25 mM borate, 5 mM CaCl₂; pH 8.5. The high pI proteases remained in the material not binding to the matrix.

The high pI protease was further purified by affinity chromatography on an HPLC column.

The protease preparation thus obtained contained only the high pI protease. As judged by SDS-PAGE the protease comprised approximately 90% of the total protein content. The pI of the purified protease as determined by isoelectric focusing on LKB Ampholine® PAG plates (pH 3.5-9.5) was 9.3, and the MW as determined by SDS-PAGE was 32,000.

The purified protease is characterized by its ability to degrade casein (Hammarsten®), haemoglobin, skim milk, and Suc-Ala-Ala-Pro-Phe-pNA. The protease has none or very low activity towards the chromogenic substrates BAPNA and Z-Glu-pNA (N-benzyloxycarbonyl-L-glutamyl-p-nitroanilide). The protease is not inhibited by EDTA.

### EXAMPLE 3

### Wash Performance

The wash performance test was accomplished on grass juice soiled cotton at 20°C, isothermally for 10 minutes.

2.0 g/l of a commercial American type powder detergent were used. The detergent was dissolved in approx. 6° dH (German Hardness) water. The pH was 9.5. The textile/wash liquor ratio was 10 g of textile per litre of wash liquor.

Subsequent to washing, the cloths were flushed in running tap water and air-dried. The remission (%R) was determined at 460 nm.

As a measure of the wash performance differential remission, ΔR, was used being equal to the remission after wash with enzyme added, minus the remission after wash with no enzyme added.

The results of the tests are shown in Table 1.

**Table 1**

| The differential remission, ΔR, measured after wash in an American type powder detergent composition. | | | | | | | |
|---|---|---|---|---|---|---|---|
| Enzyme dosage (CPU/l) | 0.0025 | 0.005 | 0.010 | 0.05 | 0.10 | 0.20 | 0.50 |
| ΔR | 3.0 | 5.0 | 7.4 | 16.8 | 19.6 | 20.9 | 21.5 |

As indicated by the differential remission values, the protease of the invention has good washing ability.

## Claims

1. A detergent composition comprising a protease obtainable from a strain of the genus Metarrhizium, wherein said protease has activity towards N-succinyl-L-alanyl-L-alanyl-L-prolyl-L-phenylalanyl-p-nitroanilide, a pI > 8.0 and a molecular weight of approximately 32 kD and one or more enzymes selected from the group of amylases, lipases, cellulases, oxidases, and peroxidases

2. The detergent composition according to claim 1, wherein the protease is obtainable from a strain of the species M. anisopliae (Metschn.) Sorokin.

3. The detergent composition according to claim 2, wherein the protease is obtainable from the strain M. anisopliae (Metschn.) Sorokin; DSM No. 6592, or a mutant of this strain.

4. The detergent composition according to any of claims 1-3, wherein the protease has immunochemical properties identical to those of a protease derived from the strain M. anisopliae (Metschn.) Sorokin; DSM No. 6592.

5. The detergent composition according to any of claims 1-4, in which the protease has a pI of approximately 9.2-9.3, as measured on LKB Ampholine® PAG plates (pH 3.5-9.5),

6. Use of a protease obtainable from a strain of the genus Metarrhizium having activity towards N-succinyl-L-alanyl-L-alanyl-L-prolyl-L-phenylalanyl-p-nitroanilide, a pI > 8.0 and a molecular weight of approximately 32 kD as a detergent additive provided in the form of a non-dusting granulate or a stabilized liquid.

7. The use according to claim 6 of a protease obtainable from a strain of the species M. anisopliae (Metschn.) Sorokin.

8. The use according to claim 7, of a protease obtainable from the strain M. anisopliae (Metschn.) Sorokin; DSM No. 6592, or a mutant of this strain.

9. The use according to any of claims 6-8 of a protease having immunochemical properties identical or partially identical to those of a protease derived from the strain M. anisopliae (Metschn.) Sorokin; DSM No. 6592.

10. The use according to any of claims 6-9, in which the protease has a pI of approximately 9.2-9.3, as measured on LKB Ampholine® PAG plates (pH 3.5-9.5).

11. The use according to any of claims 6-10, wherein the additive further comprises one or more other enzymes selected from the group of amylases, lipases, cellulases, oxidases, and peroxidases.

12. Use of a protease obtainable from a strain of the genus Metarrhizium as detergent enzyme in a washing process, wherein said protease has activity towards N-succinyl-L-alanyl-L-alanyl-L-prolyl-L-phenylalanyl-p-nitroanilide, a pI > 8.0 and a molecular weight of approximately 32 kD.

13. The use according to claim 12, wherein the protease is obtainable from a strain of the species M. anisopliae (Metschn.) Sorokin.

14. The use according to claim 13, wherein the protease is obtainable from the strain M. anisopliae (Metschn.) Sorokin; DSM No. 6592, or a mutant of this strain.

15. The use according to any of claims 12-14, wherein the protease has immunochemical properties identical or partially identical to those of a protease derived from the strain M. anisopliae (Metschn.) Sorokin; DSM No. 6592.

16. The use according to any of claims 12-15, wherein the protease has a pI of approximately 9.2-9.3, as measured on LKB Ampholine® PAG plates (pH 3.5-9.5).

17. The use according to any of claims 12-16, which use further comprises one or more other enzymes selected from the group of amylases, lipases, cellulases, oxidases, and peroxidases.

## Patentansprüche

1. Detergens-Zusammensetzung, welche eine Protease, die aus einem Stamm der Gattung Metarrhizium erhältlich ist, wobei die Protease Aktivität gegenüber N-Succinyl-L-alanyl-L-alanyl-L-prolyl-L-phenylalanyl-p-nitroanilid, einen pI-Wert > 8,0 und ein Molekulargewicht von etwa 32 kD aufweist, und ein oder mehrere Enzym(e), ausgewählt aus der Gruppe von Amylasen, Lipasen, Cellulasen, Oxidasen und Peroxidasen, umfaßt.

2. Detergens-Zusammensetzung nach Anspruch 1, worin die Protease aus einem Stamm der Spezies M. anisopliae (Metschn.) Sorokin erhältlich ist.

3. Detergens-Zusammensetzung nach Anspruch 2, worin die Protease aus dem Stamm M. anisopliae (Metschn.) Sorokin, DSM Nr. 6592 oder einer Mutante dieses Stammes erhältlich ist.

4. Detergens-Zusammensetzung nach irgendeinem der Ansprüche 1-3, worin die Protease immunchemische Eigenschaften aufweist, welche mit denjenigen einer Protease, die aus dem Stamm M. anisopliae (Metschn.) Sorokin, DSM Nr. 6592 erhalten wurde, identisch sind.

5. Detergens-Zusammensetzung nach irgendeinem der Ansprüche 1-4, worin die Protease einen pI-Wert von etwa 9,2-9,3, wie auf LKB Ampholine®-PAG-Platten (pH 3,5-9,5) gemessen, aufweist.

6. Verwendung einer Protease, die aus einem Stamm der Gattung Metarrhizium erhältlich ist, Aktivität gegenüber N-Succinyl-L-alanyl-L-alanyl-L-prolyl-L-phenylalanyl-p-nitroanilid, einen pI-Wert 8,0 und ein Molekulargewicht von etwa 32 kD aufweist, als Detergens-Additiv, das in Form eines staubfreien Granulats oder einer stabilisierten Flüssigkeit bereitgestellt wird.

7. Verwendung nach Anspruch 6 einer Protease, die aus einem Stamm der Spezies M. anisopliae (Metschn.) Sorokin erhältlich ist.

8. Verwendung nach Anspruch 7 einer Protease, die aus dem Stamm M. anisopliae (Metschn.) Sorokin, DSM Nr. 6592 oder einer Mutante dieses Stammes erhältlich ist.

9. Verwendung nach irgendeinem der Ansprüche 6-8 einer Protease mit im_ munchemischen Eigenschaften, welche mit denjenigen einer Protease, die aus dem Stamm M. anisopliae (Metschn.) Sorokin, DSM Nr. 6592 erhalten wurde, identisch oder teilweise identisch sind.

10. Verwendung nach irgend einem der Ansprüche 6-9, worin die Protease einen pI-Wert von etwa 9,2-9,3, wie auf LKB Ampholine®-PAG-Platten (pH 3,5-9,5) gemessen, aufweist.

11. Verwendung nach irgendeinem der Ansprüche 6-10, worin das Additiv ferner ein oder mehrere weitere(s) Enzym(e), ausgewählt aus der Gruppe von Amylasen, Lipasen, Cellulasen, Oxidasen und Peroxidasen, umfaßt.

12. Verwendung einer Protease, die aus einem Stamm der Gattung Metarrhizium erhältlich ist, als Detergens-Enzym in einem Waschverfahren, worin die Protease Aktivität gegenüber N-Succinyl-L-alanyl-L-alanyl-L-prolyl-L-phenylalanyl-p-nitroanilid, einen pI-Wert > 8,0 und ein Molekulargewicht von etwa 32 kD aufweist.

13. Verwendung nach Anspruch 12, worin die Protease aus einem Stamm der Spezies M. anisopliae (Metschn.) Sorokin erhältlich ist.

14. Verwendung nach Anspruch 13, worin die Protease aus dem Stamm M. anisopliae (Metschn.) Sorokin, DSM Nr. 6592 oder einer Mutante dieses Stammes erhältlich ist.

15. Verwendung nach irgendeinem der Ansprüche 12-14, worin die Protease immunchemische Eigenschaften aufweist, welche mit denjenen einer Protease, die aus dem Stamm M. anisopliae (Metschn.) Sorokin, DSM Nr. 6592 erhalten wurde, identisch oder teilweise identisch sind.

16. Verwendung nach irgendeinem der Ansprüche 12-15, worin die Protease einen pI-Wert von etwa 9,2-93, wie gemessen auf LKB Ampholine®-PAG-Platten (pH 3,5-9,5), aufweist.

17. Verwendung nach irgendeinem der Ansprüche 12-16, welche Verwendung ferner ein oder mehrere weitere(s) Enzym(e), ausgewählt aus der Gruppe aus Amylasen, Lipasen, Cellulasen, Oxidasen und Peroxidasen, umfaßt.

## Revendications

1. Composition détergente comprenant une protéase pouvant être obtenue à partir d'une souche du genre Metarrhizium, dans laquelle ladite protéase a une activité vis-à-vis du N- succinyl-L-alanyl-L-alanyl-L-prolyl-L-phénylalanyl-p-nitroanilide, un pI supérieur à 8,0 et une masse moléculaire d'environ 32 kD, et une ou plusieurs enzymes choisies dans l'ensemble comprenant les amylases, les lipases, les cellulases, les oxydases et les peroxydases.

2. Composition détergente selon la revendication 1, dans laquelle la protéase peut être obtenue à partir d'une souche de l'espèce M. anisopliae (Metschn.) Sokorin.

3. Composition détergente selon la revendication 2, dans laquelle la protéase peut être obtenue à partir de la souche M. anisopliae (Metschn.) Sokorin ; DSM n° 6592, ou un mutant de cette souche.

4. Composition détergente selon l'une quelconque des revendications 1 à 3, dans laquelle la protéase a des propriétés immunochimiques identiques à celle d'une protéase qui dérive de la souche M. anisopliae (Metschn.) Sokorin ; DSM n° 6592.

5. Composition détergente selon l'une quelconque des revendications 1 à 4, dans laquelle la protéase a un pI d'environ 9,2-9,3, tel que mesuré sur des plaques LKB Ampholine® PAG.

6. Utilisation, en tant qu'additif pour détergents se présentant sous forme d'un granulé ne formant pas de poussière ou d'un liquide stabilisé, d'une protéase pouvant être obtenue à partir d'une souche du genre Metarrhizium ayant une activité vis-à-vis du N-succinyl-L-alanyl-L-alanyl-L-prolyl-L-phénylalanyl-p-nitroanilide, un pI supérieur à 8,0 et une masse moléculaire d'environ 32 kD.

7. Utilisation selon la revendication 6 d'une protéase pouvant être obtenue à partir d'une souche de l'espèce M. anisopliae (Metschn.) Sokorin.

8. Utilisation selon la revendication 6 d'une protéase pouvant être obtenue à partir de la souche de l'espèce M. anisopliae (Metschn.) Sokorin ; DSM n° 6592, ou d'un mutant de cette souche.

9. Utilisation selon l'une quelconque des revendications 6 à 8 d'une protéase ayant des propriétés immunochimiques identiques ou partiellement identiques à celle d'une protéase qui dérive de la souche M. anisopliae (Metschn.) Sokorin ; DSM n° 6592.

10. Utilisation selon l'une quelconque des revendications 6 à 9, pour laquelle la protéase a un pI d'environ 9,2-9,3, tel que mesuré sur des plaques LKB Amphaline® PAG (pH 3,5-9,5).

11. Utilisation selon l'une quelconque des revendications 6 à 10, pour laquelle l'additif comprend en outre une ou plusieurs autres enzymes choisies dans l'ensemble comprenant les amylases, les lipases, les cellulases, les oxydases et les peroxydases.

12. Utilisation d'une protéase pouvant être obtenue à partir d'une souche du genre Metarrhizium, en tant qu'enzyme pour détergents dans une opération de lavage, pour laquelle ladite protéase a une activité vis-à-vis du N-succinyl-L-alanyl-L-alanyl-L-prolyl-L-phénylalanyl-p-nitroanilide, un pI supérieur à 8,0 et une masse moléculaire d'environ 32 kD.

13. Utilisation selon la revendication 12, pour laquelle la protéase peut être obtenue à partir d'une souche de l'espèce M. anisopliae (Metschn.) Sokorin.

14. Utilisation selon la revendication 13, pour laquelle la protéase peut être obtenue à partir d'une souche de l'espèce M. anisopliae (Metschn.) Sokorin, DSM n° 6592, ou d'un mutant de cette souche.

15. Utilisation selon l'une quelconque des revendications 12 à 14, pour laquelle la protéase a des propriétés immunochimiques identiques ou partiellement identiques à celles d'une protéase qui dérive de la souche de l'espèce M. anisopliae (Metschn.) Sokorin, DSM n° 6592.

16. Utilisation selon l'une quelconque des revendications 12 à 15, pour laquelle la protéase a un pI d'environ 9,2-9,3, tel que mesuré sur des plaques LKB Ampholine® PAG (pH 3,5-9,5).

17. Utilisation selon l'une quelconque des revendications 12 à 16, laquelle utilisation comprend en outre une ou plusieurs enzymes choisies dans l'ensemble comprenant les amylases, les lipases, les cellulases, les oxydases et les peroxydases.
